# FASCICULE DE BREVET EUROPEEN

(11) **EP 1 680 504 B1**
(45) Date de publication et mention de la délivrance du brevet: **19.10.2011**
(21) Numéro de dépôt: 04805397.9
(22) Date de dépôt: 05.11.2004
(51) Int. Cl.: C12N 9/02, C12P 13/00, C12P 7/00, C12P 33/00

(54) **SOUCHES DE MICROORGANISMES OPTIMISEES POUR DES VOIES DE BIOSYNTHESE CONSOMMATRICES DE NADPH**
OPTIMIERTE MIKROORGANISMENSTÄMME FÜR NADPH VERBRAUCHENDE BIOSYNTHESEWEGE
OPTIMISED MICRO-ORGANISM STRAINS FOR NADPH-CONSUMING BIOSYNTHETIC PATHWAYS

(30) Priorité: 06.11.2003 FR 0313056
(43) Date de publication de la demande: 19.07.2006
(73) Titulaire: Metabolic Explorer, 63360 Saint Beauzire (FR)
(72) Inventeur: BESTEL-CORRE, Gwénaëlle, F-63360 Saint Beauzire (FR); BOISART, Cédric, 63400 Chamallieres (FR); CHATEAU, Michel, 63200 Riom (FR); GONZALEZ, Benjamin, F-63200 Riom (FR); SOUCAILLE, Philippe, F-31450 Deyme (FR); FIGGE, Rainer, F-63200 Riom (FR); ZINK, Olivier, FR / F-68100 MULHOUSE (FR)
(74) Mandataire: Tetaz, Franck Claude Edouard
(86) Numéro de dépôt international: PCT/FR2004/002848
(87) Numéro de publication internationale: WO 2005/047498

(56) Documents cités:
- EP-A- 1 170 376
- WO-A-98/02552
- KERVINEN MARKO ET AL: "A pair of membrane-embedded acidic residues in the NuoK subunit of Escherichia coli NDH-1, a counterpart of the ND4L subunit of the mitochondrial complex I, are required for high ubiquinone reductase activity." BIOCHEMISTRY, vol. 43, no. 3, 27 janvier 2004 (2004-01-27), pages 773-781, XP002324463 ISSN: 0006-2960
- CALHOUN MELISSA W ET AL: "Demonstration of separate genetic loci encoding distinct membrane-bound respiratory NADH dehydrogenases in Escherichia coli" JOURNAL OF BACTERIOLOGY, vol. 175, no. 10, 1993, pages 3013-3019, XP009046383 ISSN: 0021-9193
- BERTSOVA YULIA V ET AL: "Noncoupled NADH:Ubiquinone oxidoreductase of Azotobacter vinelandii is required for diazotrophic growth at high oxygen concentrations" JOURNAL OF BACTERIOLOGY, vol. 183, no. 23, décembre 2001 (2001-12), pages 6869-6874, XP002324518 ISSN: 0021-9193
- PIEULLE L ET AL: "The gene encoding the NdhH subunit of type 1 NAD(P)H dehydrogenase is essential to survival of synechocystis PCC6803." FEBS LETTERS. 29 DEC 2000, vol. 487, no. 2, 29 décembre 2000 (2000-12-29), pages 272-276, XP004337974 ISSN: 0014-5793
- MOLENAAR DOUWE ET AL: "Functions of the membrane-associated and cytoplasmic malate dehydrogenases in the citric acid cycle of Corynebacterium glutamicum" JOURNAL OF BACTERIOLOGY, WASHINGTON, DC, US, vol. 182, no. 24, décembre 2000 (2000-12), pages 6884-6891, XP002182880 ISSN: 0021-9193
- FRERICHS-DEEKEN URSULA ET AL: "Functional expression of the quinoline 2-oxidoreductase genes (qorMSL) in Pseudomonas putida KT2440 pUF1 and in P. putida 86-1 deltaqor pUF1 and analysis of the Qor proteins." EUROPEAN JOURNAL OF BIOCHEMISTRY / FEBS, (2003 APR) 270 (7) 1567-77., avril 2003 (2003-04), XP002274625
- SAUER UWE ET AL: "The soluble and membrane-bound transhydrogenases UdhA and PntAB have divergent functions in NADPH metabolism of Escherichia coli." THE JOURNAL OF BIOLOGICAL CHEMISTRY. UNITED STATES 20 FEB 2004, vol. 279, no. 8, 20 février 2004 (2004-02-20), pages 6613-6619, XP002274624 ISSN: 0021-9258
- YAMAGUCHI MUTSUO ET AL: "Proton-translocating nicotinamide nucleotide transhydrogenase of Escherichia coli: Involvement of aspartate 213 in the membrane-intercalating domain of the beta subunit in energy transduction" JOURNAL OF BIOLOGICAL CHEMISTRY, vol. 270, no. 28, 1995, pages 16653-16659, XP002324466 ISSN: 0021-9258
- MORITA TEPPEI ET AL: "Accumulation of glucose 6-phosphate or fructose 6-phosphate is responsible for destabilization of glucose transporter mRNA in Escherichia coli." JOURNAL OF BIOLOGICAL CHEMISTRY, vol. 278, no. 18, 2 mai 2003 (2003-05-02), pages 15608-15614, XP002274626 ISSN: 0021-9258
- NYUNOYA, HIROSHI ET AL: "Effects of sugars and polyols on basidiocarp formation in a phosphoglucose isomerase mutant of Coprinus cinereus" CANADIAN JOURNAL OF MICROBIOLOGY (1984), 30(1), 45-51, 1984, XP009027484
- MARX ACHIM ET AL: "Metabolic phenotype of phosphoglucose isomerase mutants of Corynebacterium glutamicum." JOURNAL OF BIOTECHNOLOGY, (2003 SEP 4) 104 (1-3) 185-97., 4 septembre 2003 (2003-09-04), XP002263663
- BOLES E ET AL: "THE ROLE OF THE NAD-DEPENDENT GLUTAMATE GEHYDROGENASE IN RESTORING GROWTH ON GLUCOSE OF A SACCHAROMYCES CEREVISIAE PHOSPHOGLUCOSE ISOMERASE MUTANT" EUROPEAN JOURNAL OF BIOCHEMISTRY, BERLIN, DE, vol. 217, no. 1, 1 octobre 1993 (1993-10-01), pages 469-477, XP000960373 ISSN: 0014-2956

### SEQUENCE LISTING

<110> METABOLIC EXPLORER
<120> Souches de microorganismes optimisées pour des voies de biosynthèses consommatrices de NADPH
<130> D21726
<150> FR 0313056
<151> 2003-11-06
<160> 38
<170> PatentIn version 3.1
<210> 1
   <211> 100
   <212> DNA
   <213> oligonucleotide synthétique
<400> 1
   ggtgcgcgcg tcgcagttat cgagcgttat caaaatgttg gcggcggttg cacccactgg     60
   ggcaccatcc cgtcgaaagc catatgaata tcctccttag                          100
<210> 2
   <211> 100
   <212> DNA
   <213> oligonucleotide synthétique
<400> 2
   cccagaatct cttttgtttc ccgatggaac aaaattttca gcgtgcccac gttcatgccg 60
   acgatttgtg cgcgtgccag tgtaggctgg agctgcttcg 100
<210> 3
   <211> 30
   <212> DNA
   <213> oligonucleotide synthétique
<400> 3
   ggccgctcag gatatagcca gataaatgac 30
<210> 4
   <211> 30
   <212> DNA
   <213> oligonucleotide synthétique
<400> 4
   gcgggatcac tttactgcca gcgctggctg 30
<210> 5
   <211> 100
   <212> DNA
   <213> oligonucleotide synthétique
<400> 5
   ggtggcccgg aagtacttca agccgtagag ttcactcctg ccgatccggc ggagaatgaa 60
   atccaggtcg aaaataaagc catatgaata tcctccttag 100
<210> 6
   <211> 100
   <212> DNA
   <213> oligonucleotide synthétique
<400> 6
   cgcccggctt tccagaatct catgcgcacg ctgcgcatcc ttcagcggat atttctgctg 60
   ctcggcgaca tcgaccttaa tgtaggctgg agctgcttcg 100
<210> 7
   <211> 30
   <212> DNA
   <213> oligonucleotide synthétique
<400> 7
   cgcccaacac cgactgctcc gcttcgatcg 30
<210> 8
   <211> 30
   <212> DNA
   <213> oligonucleotide synthétique
<400> 8
   cagcgttatg accgctggcg ttactaaggg 30
<210> 9
   <211> 43
   <212> DNA
   <213> oligonucleotide synthétique
<400> 9
   acgtacgtgg catatgtcag ttttcgtttc aggtgctaac ggg 43
<210> 10
   <211> 44
   <212> DNA
   <213> oligonucleotide synthétique
<400> 10
   acgtacctgc agttatattc tgccctcaaa ttttaaaatt tggg 44
<210> 11
   <211> 100
   <212> DNA
   <213> oligonucleotide synthétique
<400> 11
   ccaacgcaga ccgctgcctg gcaggcacta cagaaacact tcgatgaaat gaaagacgtt 60
   acgatcgccg atctttttgc tgtaggctgg agctgcttcg 100
<210> 12
   <211> 100
   <212> DNA
   <213> oligonucleotide synthétique
<400> 12
   gcgccacgct ttatagcggt taatcagacc attggtcgag ctatcgtggc tgctgatttc 60
   tttatcatct ttcagctctg catatgaata tcctccttag 100
<210> 13
   <211> 30
   <212> DNA
   <213> oligonucleotide synthétique
<400> 13
   gcggggcggt tgtcaacgat ggggtcatgc 30
<210> 14
   <211> 30
   <212> DNA
   <213> oligonucleotide synthétique
<400> 14
   cggtatgatt tccgttaaat tacagacaag 30
<210> 15
   <211> 102
   <212> DNA
   <213> oligonucleotide synthétique
<400> 15
   cgcgcgagac tcgctctgct tatctcgccc ggatagaaca agcgaaaact tcgaccgttc 60
   atcgttcgca gttggcatgc ggtgtaggct ggagctgctt cg 102
<210> 16
   <211> 98
   <212> DNA
   <213> oligonucleotide synthétique
<400> 16
   cgcaaggcgc tgaataattc acgtcctgtt cccacgcgtg acgcgctcag gtcaggaatg 60
   tgcggttcgc gagcagccca tatgaatatc ctccttag 98
<210> 17
   <211> 29
   <212> DNA
   <213> oligonucleotide synthétique
<400> 17
   gggtagactc cattactgag gcgtgggcg 29
<210> 18
   <211> 24
   <212> DNA
   <213> oligonucleotide synthétique
<400> 18
   ccccggaatc agaggaatag tccc 24
<^10> 19
   <211> 100
   <212> DNA
   <213> oligonucleotide synthétique
<400> 19
<210> 20
   <211> 100
   <212> DNA
   <213> oligonucleotide synthétique
<400> 20
   ttcgcgcagt ccagccagtc acctttgaac ggacgcttca tgttttcgat agcgtcgatg 60
   atgtcgtggt gaaccagctg catatgaata tcctccttag 100
<210> 21
   <211> 27
   <212> DNA
   <213> oligonucleotide synthétique
<400> 21
   cgcacgcggc agtcagggcc gacccgc 27
<210> 22
   <211> 27
   <212> DNA
   <213> oligonucleotide synthétique
<400> 22
   ccctacgccc cacttgttca tcgcccg 27
<^10> 23
   <211> 99
   <212> DNA
   <213> oligonucleotide synthétique
<400> 23
<210> 24
   <211> 100
   <212> DNA
   <213> oligonucleotide synthétique
<400> 24
   gcgggaaagg taagcgtaaa ttttttgcgt atcgtcatgg gagcacagac gtgttccctg 60
   attgagtgtg gctgcactcc catatgaata tcctccttag 100
<210> 25
   <211> 30
   <212> DNA
   <213> oligonucleotide synthétique
<400> 25
   tggcaggatc atccatgaca gtaaaaacgg 30
<210> 26
   <211> 26
   <212> DNA
   <213> oligonucleotide synthétique
<400> 26
   gccggttgca ctttgggtaa gccccg 26
<210> 27
   <211> 43
   <212> DNA
   <213> oligonucleotide synthétique
<400> 27
   acgtacgtcc cccgggaaaa atgtcagttt tcgtttcagg tgc 43
<210> 28
   <211> 44
   <212> DNA
   <213> oligonucleotide synthétique
<400> 28
   acgtacgggc ccttatattc tgccctcaaa ttttaaaatt tggg 44
<210> 29
   <211> 100
   <212> DNA
   <213> oligonucleotide synthétique
<400> 29
   ccaacgcaga ccgctgcctg gcaggcacta cagaaacact tcgatgaaat gaaagacgtt 60
   acgatcgccg atctttttgc tgtaggctgg agctgcttcg 100
<210> 30
   <211> 100
   <212> DNA
   <213> oligonucleotide synthétique
<400> 30
   gcgccacgct ttatagcggt taatcagacc attggtcgag ctatcgtggc tgctgatttc 60
   tttatcatct ttcagctctg catatgaata tcctccttag 100
<210> 31
   <211> 30
   <212> DNA
   <213> oligonucleotide synthétique
<400> 31
   gcggggcggt tgtcaacgat ggggtcatgc 30
<210> 32
   <211> 30
   <212> DNA
   <213> oligonucleotide synthétique
<400> 32
   cggtatgatt tccgttaaat tacagacaag 30
<210> 33
   <211> 82
   <212> DNA
   <213> oligonucleotide synthétique
<400> 33
   atgcaatctc aagattcatg ctacggtgtt gcattcagat ctatcatcac aaatgatgaa 60
   aagcttcgta cgctgcaggt cg 82
<210> 34
   <211> 83
   <212> DNA
   <213> oligonucleotide synthétique
<400> 34
   tttgttttca gcggctaaag cggctacctc agctctcaac tttaatctac cggacaggat 60
   gggccactag tggatctgat atc 83
<210> 35
   <211> 22
   <212> DNA
   <213> oligonucleotide synthétique
<400> 35
   gctttcttag aagctacctc cg 22
<210> 36
   <211> 22
   <212> DNA
   <213> oligonucleotide synthétique
<400> 36
   gaaccgacaa gaccaacaat gg 22
<210> 37
   <211> 20
   <212> DNA
   <213> oligonucleotide synthétique
<400> 37
   cagttgtaca ctttggaccc 20
<210> 38
   <211> 22
   <212> DNA
   <213> oligonucleotide synthétique
<400> 38
   gatcagcacc agtcaaagaa cc 22

## Description

Le NADP (nicotinamide adénine dinucléotide phosphate) participe sous sa forme réduite, NADPH, aux réactions intracellulaires d'oxydoréductions impliquant des enzymes à activité déshydrogénase ou réductase.

La présente invention concerne des souches de microorganismes optimisées pour la production par biotransformation, de molécules ayant des voies de biosynthèse consommatrices de NADPH. Les souches selon l'invention sont utilisables dans des procédés de biotransformation consommateurs de NADPH. Les souches définies selon l'invention peuvent être procaryotiques ou eucaryotiques. Dans un mode de réalisation préféré, ladite souche procaryotique est une souche *d'Escherichia coli.* Dans un autre mode de réalisation, ladite souche eucaryotique est une souche de *Saccharomyces,* en particulier *S. cerevisiae.*

La présente invention concerne également un procédé de préparation de molécules par biotransformation comprenant la culture dans un milieu approprié d'une souche optimisée selon l'invention, ladite souche optimisée comprenant également les éléments génétiques nécessaires à la préparation de ladite molécule.

Les procédés de biotransformation ont été développés pour permettre la production de molécules en grande quantité à des coûts faibles, tout en permettant également la valorisation de différents sous-produits industriels ou de l'agriculture.

Pour produire des molécules d'intérêt par biotransformation *in vivo* on distinguera deux grandes approches :
- d'une part la fermentation qui permet la production de molécules par un microorganisme à partir d'une source de carbone simple (*e.g.* WO0102547 qui décrit la production de lysine par fermentation de *C. glutamicum* en présence de glucose),
- d'autre part la bioconversion par un microorganisme d'un co-substrat donné en une molécule d'intérêt (e.g. WO0012745 qui décrit la production de dérivés R-pipéridine, WO0068397 qui décrit la production de tagatose). Le co-substrat est non assimilable ; il est différent de la source de carbone qui est utilisée seulement pour produire la biomasse et le NADPH nécessaire à la bioconversion.

L'amélioration d'un procédé de biotransformation peut porter sur différents facteurs comme la température, l'oxygénation, la composition du milieu, le procédé de récupération, etc. On peut aussi envisager de modifier le microorganisme de telle sorte que la production de la molécule d'intérêt et/ou son excrétion soit augmentée.

Dans le cadre d'une fermentation on s'attachera par exemple à optimiser la voie de biosynthèse, par exemple en modifiant la régulation des gènes ou en modifiant les gènes afin de modifier les caractéristiques des enzymes impliquées, ou encore en optimisant la régénération des cofacteurs.

Dans le cadre de la bioconversion on s'attachera davantage à réduire la formation de co-produits et à optimiser la régénération de cofacteurs impliqués dans la ou les étapes de bioconversion.

Parmi les cofacteurs impliqués dans les biotransformations, le NADPH prend une part importante notamment pour la production des acides aminés (*e.g.* arginine, proline, isoleucine, méthionine, lysine), de vitamines (*e.g.* panthoténate, phylloquinone, tocophérol), de molécules aromatiques (e.g. WO401564), de polyols (*e.g.* xylitol), de polyamines (*e.g.* spermidine). d'hydroxyesters (e.g. éthyl-4-chloro-3-hydroxybutyrate) ou d'autres molécules à haute valeur ajoutée.

La présente invention concerne donc une souche de microorganismes optimisée pour la production de molécules ayant des voies de biosynthèse consommatrices de NADPH telle que définie dans la revendication 1.

Au lieu d'essayer d'optimiser pour chaque biotransformation le ratio NADPH/NADP⁺ dans le microorganisme, les inventeurs ont opté pour la production de microorganismes modifiés afin d'obtenir différents ratios NADPH/NADP⁺, lesdits microorganismes modifiés étant ensuite employés pour réaliser les biotransformations consommatrices de NADPH.

Par souche de microorganismes, on entend selon l'invention un ensemble de microorganismes d'une même espèce comprenant au moins un microorganisme de ladite espèce. Ainsi, les caractéristiques décrites pour la souche s'appliquent à chacun des microorganismes de ladite souche. De même, les caractéristiques décrites pour l'un des microorganismes de la souche s'appliqueront à l'ensemble desdits microorganismes la composant.

Parmi les microorganismes optimisés selon l'invention, on citera les bactéries et les levures, les champignons filamenteux et notamment les bactéries et les levures des espèces suivantes : *Aspergillus sp., Bacillus sp., Brevibacterium sp., Clostridium sp., Corynebacterium sp., Escherichia sp., Gluconobacter sp., Penicillium sp., Pichia sp., Pseudomonas sp., Rhodococcus sp., Saccharomyces sp., Streptomyces sp., Xanthomonas sp., Candida sp.*

Le principe de l'optimisation du ratio NADPH/NADP⁺ est décrit ci-après pour *E. coli* et *S. cerevisiae.* Le même principe peut être appliqué de manière similaire à tous les microorganismes cultivés en conditions aérobies.

Le principe de l'optimisation du ratio NADPH/NADP⁺ consiste à limiter les activités enzymatiques impliquées dans l'oxydation du NADPH, et à favoriser les activités enzymatiques permettant la réduction du NADP⁺. On limite les activités enzymatiques impliquées dans l'oxydation du NADPH en diminuant, et plus particulièrement en inactivant, de telles activités, notamment les activités de type quinone oxidoréductase et/ou transhydrogénase soluble. On favorise les activités enzymatiques permettant la réduction du NADP⁺ en imposant le flux de carbone via le cycle des pentoses phosphate et/ou en modifiant la spécificité de cofacteur d'au moins une enzyme de telle sorte qu'elle utilise le NADP préférentiellement au NAD, son cofacteur habituel.

Les souches optimisées selon l'invention sont obtenues par biologie moléculaire. L'homme du métier connaît les protocoles permettant de modifier le caractère génétique des microorganismes. Les techniques de transformation sont documentées et sont à la portée de l'homme du métier (Sambrook et al., 1989 Molecular cloning : a laboratory manual. 2nd Ed. Cold Spring Harbor Lab., Cold Spring Harbor, New York.).

Les procédés permettant de limiter une activité enzymatique consistent à modifier le gène permettant son expression par un moyen approprié, par exemple en apportant une ou plusieurs mutation(s) dans la partie codante du gène concerné, ou en modifiant la région promotrice, notamment en la remplaçant par une séquence permettant de diminuer l'expression du gène.

Les procédés permettant d'inactiver une activité enzymatique consistent à inactiver le produit d'expression du gène concerné par un moyen approprié, ou bien à inhiber l'expression du gène concerné, ou encore à déléter au moins une partie du gène concerné, de manière à ce que soit son expression n'ait pas lieu (par exemple délétion d'une partie ou de l'ensemble de la région promotrice nécessaire à son expression), soit le produit d'expression ait perdu sa fonction (par exemple délétion dans la partie codante du gène concerné).

De manière préférentielle, la délétion d'un gène comprend la suppression de l'essentiel dudit gène, et le cas échéant son remplacement par un gène marqueur de sélection permettant de faciliter l'identification, l'isolement et la purification des souches optimisées selon l'invention.

L'inactivation d'un gène chez *E. coli* se fait préférentiellement par recombinaison homologue (Datsenko, K.A. ; Wanner, B.L. (2000) One-step inactivation of chromosomal genes in Escherichia coli K-12 using PCR products. Proc. Natl. Acad. Sci. USA 97: 6640-6645). Le principe d'un protocole en est rappelé brièvement : on introduit dans la cellule un fragment linéaire, obtenu *in vitro,* comprenant les deux régions flanquant le gène, et au moins un gène de sélection entre ces deux régions (généralement un gène de résistance à un antibiotique), ledit fragment linéaire présentant donc un gène inactivé. On sélectionne les cellules ayant subi un événement de recombinaison et ayant intégré le fragment introduit par étalement sur milieu sélectif. On sélectionne ensuite les cellules ayant subi un événement de double recombinaison, dans lesquelles le gène natif a été remplacé par le gène inactivé. Ce protocole peut être amélioré en utilisant des systèmes de sélections positive et négative, afin d'accélérer la détection des événements de double recombinaisons.

L'inactivation d'un gène chez *S.cerevisiae* se fait également préférentiellement par recombinaison homologue (Baudin et al., Nucl. Acids Res. 21, 3329-3330, 1993; Wach et al., Yeast 10, 1793-1808, 1994; Brachmann et al., Yeast. 14 :115-32, 1998).

Les procédés permettant de favoriser une activité enzymatique consistent à stabiliser le produit d'expression du gène concerné par un moyen approprié par exemple en diminuant sa sensibilité à des effecteurs allostériques, ou bien à augmenter l'expression du dit gène de manière à augmenter la quantité d'enzyme.

La surexpression d'un gène peut être effectuée par changement du promoteur de ce gène *in situ,* par un promoteur fort ou inductible. De façon alternative, on introduit, dans la cellule, un plasmide réplicatif (simple ou multicopies) dans lequel le gène que l'on désire surexprimer est sous le contrôle du promoteur adéquat. Dans le cas de modification *d'Escherichia coli,* on pourra par exemple utiliser les promoteurs P*lac-*o, P*trc-*o, p*tac-*o, trois promoteurs forts bactériens pour lesquels l'opérateur lac *(lac*O*)* a été délété pour les rendre constitutifs. Dans le cas de modifications de *Saccharomyces cerevisiae,* on pourra par exemple utiliser les promoteurs P*pgk,* P*adh*1*,* P*gal*1*,* P*gal*10*.*

Les procédés permettant de modifier la spécificité de cofacteur d'une enzyme de telle sorte qu'elle utilise le NADP préférentiellement au NAD, consistent à modifier la séquence du gène permettant l'expression de cette enzyme (Bocanegra, J.A. Scrutton, N.S. ; Perham, R.N. (1993) Creation of an NADP-dependent pyruvate dehydrogenase multienzyme complex by protein engineering. Biochemistry 32 : 2737-2740).

Les souches optimisées selon l'invention (*i.e*. capacité accrue de réduction du NADP⁺) comprennent l'atténuation voire l'inactivation, d'une ou plusieurs activité(s) enzymatique(s) oxydant le NADPH, et en particulier, des activités de type quinone oxidoréductase et/ou transhydrogénase soluble.

On citera pour exemples d'enzymes oxydant le NADPH et sans que cette liste soit limitative les activités et les gènes suivants :

| **Activités enzymatiques** | **Numéro EC** | **Gènes *E. coli*** | **Gènes *S. cerevisiae*** |
|---|---|---|---|
| Alcool déshydrogénase | 1.1.1.2 | *yahK* | ADH6 |
| Aldose réductase | 1.1.1.21 | | GRE3 |
| Shikimate déshydrogénase | 1.1.1.25 | *aroE* | ARO1 |
| Méthylglyoxal réductase | 1.1.1.78 | | GRE2p |
| Gamma-glutamyl phosphate réductase | 1.2.1.41 | *proA* | PRO2 |
| 2,4-diénoyl coenzyme A réductase | 1.3.1.34 | *fadH* | |
| Glutamate déshydrogénase | 1.4.1.4 | *gdhA* | GDH1, GDH2 |
| Glutamate synthase | 1.4.1.13 | *gltB, gltD* | GLT1 |
| Méthylènetétrahydrofolate déshydrogénase | 1.5.1.5 | *fold* | ADE3, MIS1 |
| Transhydrogénase soluble | 1.6.1.1 | *udhA* | |
| Transhydrogénase membranaire | 1.6.1.2 | *pntA, pntB* | |
| Quinone oxidoréductase | 1.6.5.5 | *qor* | ZTA1 |
| Nitrite réductase | 1.7.1.4 | *nirB, nirD* | |
| Sulfite réductase | 1.8.1.2 | *cysl, cysJ* | |
| Stérol déméthylase | 1.14.13.70 | | ERG11 |
| 4-Hydroxy-3-méthylbut-2-ényl diphosphate réductase | 1.17.1.2 | *ispH* | |
| Flavodoxin réductase | 1.18.1.2 | *fpr* | |

Les souches optimisées selon l'invention (*i.e*. capacité accrue de réduction du NADP⁺ comprennent également des modifications permettant de favoriser une ou plusieurs activité(s) enzymatique(s) réduisant le NADP⁺, et en particulier, des modifications permettant d'imposer le flux de carbone via le cycle des pentoses phosphate, et/ou des modifications portant sur la spécificité de cofacteur d'au moins une enzyme, de telle sorte qu'elle utilise le NADP préférentiellement au NAD, son cofacteur habituel.

Les activités susceptibles d'être modifiés dans les souches optimisées selon l'invention (i.e. capacité accrue de réduction du NADP⁺) afin de favoriser une ou plusieurs activité(s) enzymatique(s) réduisant le NADP⁺, sont décrites ci-dessous :

| **Activités enzymatiques** | **Numéro EC** | **Gènes *E. coli*** | **Gènes *S. cerevisiae*** |
|---|---|---|---|
| Phosphoglucose isomérase | 5.3.1.9 | *pgi* | PGI1 |
| Phosphofructokinase | 2.7.1.11 | *pfkA, pfkB* | PFK1, PFK2 |
| Glucose 6-phosphate déshydrogénase | 1.1.1.49 | *zwf* | ZWF1 |
| 6-Phosphogluconolactonase | 3.1.1.31 | | SOL1, SOL2, SOL3, SOL4 |
| 6-Phosphogluconate déshydrogénase | 1.1.1.44 | *gnd* | GND1, GND2 |
| 6-Phosphogluconate déshydratase | 4.2.1.12 | *edd* | |
| Malate synthase | 2.3.3.9 | *aceB* | DAL7 |
| Isocitrate lyase | 4.1.3.1 | *aceA* | ICL1 |
| Isocitrate déshydrogénase | 1.1.1.42 | *icd* | IDP1, IDP2, IDP3 |
| Isocitrate déshydrogénase kinase/phosphatase | 2.7.1.116 | *aceK* | |
| Dihydrolipoamide déshydrogénase | 1.8.1.4 | *Ipd* | LPD1 |
| Glycéraldehyde-3-phosphate déshydrogénase | 1.2.1.12 | *gapA, gapC* | TDH1, TDH2, TDH3 |

Les activités enzymatiques susceptibles d'être modifiés dans les souches optimisées selon l'invention sont définies principalement par l'emploi de la dénomination de la protéine ou du gène chez *E. coli* ou *S. cerevisiae.* Cependant, cet emploi a une signification plus générale selon l'invention et englobe les activités enzymatiques correspondantes chez d'autres microorganismes. En effet en utilisant les les séquences des protéines et des gènes d'*E. coli* ou de *S. cerevisiae,* l'homme du métier est capable de déterminer les protéines et les gènes équivalents dans d'autres microorganismes qu'*E. coli* ou *S. cerevisiae.*

Les moyens d'identification des séquences homologues et de leurs pourcentages d'homologie sont bien connus de l'homme du métier, comprenant notamment le programme BLAST qui peut être utilisé à partir du site http://www.ncbi.nlm.nih.gov/BLAST/ avec les paramètres indiqués par défaut sur ce site. Les séquences obtenues peuvent alors être exploitées (e.g. alignées) en utilisant par exemple les programmes CLUSTALW (http://www.ebi.ac.uk/clustalw/) ou MULTALTN (http://prodes.toulouse.inra.fr/multalin/cgi-bin/multalin.pl), avec les paramètres indiqués par défaut sur ces sites.

Alternativement il est possible d'utiliser le programme CD-Search-(hthp://www.ncbi.nih.gov/Structure/cdd/wrpsb.cgi) qui permet d'identifier des domaines conservés dans les séquences protéiques d'*E. coli* ou de *S. cerevisiae,* et de rechercher des séquences d'autres microorganismes, présentant le ou les même(s) domaine(s). Les domaines conservés sont répertoriés dans la base de données CDD (Conserved domain database ; Marchler-Bauer A, Anderson JB, DeWeese-Scott C, Fedorova ND, Geer LY, He S, Hurwitz DI, Jackson JD, Jacobs AR, Lanczycki CJ, Liebert CA, Liu C, Madej T, Marchler GH, Mazumder R, Nikolskaya AN, Panchenko AR, Rao BS, Shoemaker BA, Simonyan V, Song JS, Thiessen PA, Vasudevan S, Wang Y, Yamashita RA, Yin JJ, Bryant SH. CDD: a curated Entrez database of conserved domain alignments. Nucleic Acids Research 31:383-387 (2003)) qui regroupe les données de type PFAM ou COG.

Les PFAM (Protein FAMilies database of alignments and hidden markov models ; hnp://www.sanger.ac.uk-/Software/Pfam/) représentent une large collection d'alignements de séquences protéiques. Chaque PFAM permet de visualiser des alignements multiples, de voir des domaines protéiques, d'évaluer la répartition entre les organismes, d'avoir accès à d'autres bases de données, de visualiser des structures connues de protéines.

Les COGs (Clusters of Orthologous Groups of proteins ; http://www.ncbi.nlm.nih.gov/COG sont obtenus en comparant les séquences protéiques issus de 43 génomes complètement séquencés représentant 30 lignées phylogénétiques majeurs. Chaque COG est défini à partir d'au moins trois lignées ce qui permet ainsi d'identifier des domaines conservés anciens.

À partir des séquences consensus identifiées par ces différentes méthodes, il est possible de dessiner des sondes oligonucléotidiques dégénérées permettant de cloner le gène correspondant chez un autre microorganisme. Ces techniques de routine de biologie moléculaire sont bien connues dans l'art et sont décrites par exemple dans Sambrook et al. (1989 Molecular cloning : a laboratory manual. 2nd Ed. Cold Spring Harbor Lab., Cold Spring Harbor, New York.).

### Exemples de gènes codant pour des protéines analogues à la transhydrogénase soluble d'E. coli codée par le gène udhA:

| **Gènes** | **Microorganismes** |
|---|---|
| *sth* | *Azotobacter vinelandii* |
| *udhA* | *Salmonella typhimurium* LT2 |
| *sthA* | *Pseudomonas aeruginosa* PA01 |
| *sth* | *Pseudomonas fluorescens* |
| *sthA* | *Pseudomonas putida* KT2440 |
| *udhA* | *Shigella flexneri* 2a str. 301 |
| *sthA* | *Vibrio cholera* |
| *sthA* | *Yersinia pestis* |

### Exemples de gènes codant pour des protéines analogues à la quinone oxidoréductase d'E. coli codée par le gène qor:

| **Gènes** | **Microorganismes** |
|---|---|
| *qor* | *Bradyrhizobium japonicum* USDA 110 |
| *qor* | *Brucella suis* 1330 |
| CC3759 | *Caulobacter crescentus* |
| mll0505 | *Mesorhizobium loti* |
| *qor* | *Mycobacterium tuberculosis* H37RV |
| *qor* | *Pseudomonas aeruginosa* |
| ZTA1 | *S. cerevisiae* |
| SPCC285.01c | *Schizosaccharomyces pombe* |
| *drgA* | *Synechocystis sp.* PCC6803 |
| *qorA* | *Staphylococcus aureus* |
| TTC0305 | *Thermus thermophilus* H B8 |
| *qor* | *Yersinia pestis* CO92 |

Les souches optimisées selon l'invention (*i.e*. capacité accrue de réduction du NADP⁺) comprennent la délétion d'au moins un gène codant pour une activité oxydant le NADPH, et en particulier, la délétion d'un gène codant pour une Quinone oxidoréductase (e.g. *qor*, ZTA1) et/ou d'un gène codant pour une activité Transhydrogénase soluble (e.g. *udhA).*

Selon un mode préférentiel de réalisation de l'invention, les gènes *udhA* et *qor* sont tous deux délétés.

Selon un mode particulier de réalisation de l'invention, les souches optimisées selon l'invention comprennent également la délétion d'un ou plusieurs gène(s) codant pour les activités Phosphoglucose isomérase (e.g. *pgi,* PG11) et/ou Phosphofructokinase (e.g. *pfkA*, PFK1).

Selon un autre mode particulier de réalisation de l'invention, les souches optimisées selon l'invention comprennent également la modification d'un ou plusieurs gène(s) codant pour les activités Dihydrolipoamide déshydrogénase *(e.g. lpd,* LPD1) et/ou Glycéraldéhyde 3-phosphate déshydrogénase (e.g. *gapA,* TDH1), la modification consistant à modifier la préférence de l'enzyme au profit du NADP au lieu du NAD, son cofacteur habituel.

Les souches selon l'invention ayant la délétion des gènes codant pour les activités Phosphoglucose isomérase et/ou Phosphofructokinase sont plus particulièrement adaptées pour les procédés de biotransformation.

Pour augmenter davantage la quantité de NADPH disponible dans les microorganismes optimisés selon l'invention, il peut être également avantageux de surexprimer au moins un gène codant pour une activité enzymatique parmi la Glucose 6-phosphate déshydrogénase (e.g. *zwf*, ZWF1), la 6-Phosphogluconolactonase (e.g. SOL1), la 6-Phosphogluconate déshydrogénase (e.g. *gnd,* GND1), l'Isocitrate déshydrogénase (e.g. *icd,* IDP1) et la Transhydrogénase membranaire (e.g. *pntA),* et/ou de déléter au moins un gène codant pour une activité enzymatique parmi la 6-Phosphogluconate déshydratase (e.g. *edd),* la Malate synthase *(e.g. aceB,* DAL7), l'Isocitrate lyase (e.g. *aceA,* ICL1) et l'Isocitrate déshydrogénase kinase/phosphatase (e.g. *aceK).*

La présente invention a également pour objet un microorganisme optimisé pour la production de NADPH telle que définie ci-dessus et ci-après, lequel comprend également, un ou plusieurs gènes codant pour des activités enzymatiques impliquées dans la biotransformation d'une molécule d'intérêt, ainsi qu'un ou plusieurs gènes marqueurs de sélection.

Ces gènes peuvent être natifs de la souche optimisée selon l'invention ou encore introduits dans la souche optimisée selon l'invention par transformation avec un vecteur approprié, soit par intégration dans le génome du microorganisme ou encore par un vecteur réplicatif, ledit vecteur approprié portant un ou plusieurs gènes codant pour lesdites enzymes impliqués dans la biotransformation de ladite molécule d'intérêt et/ou lesdits marqueurs de sélection.

Ces gènes comprennent une séquence d'acide nucléique codant pour une enzyme impliquée dans la biotransformation de la molécule d'intérêt et/ou pour un marqueur de sélection, la séquence codante étant fusionnée à des séquences promotrices efficaces dans la cellule procaryote et/ou eucaryote choisie pour la biotransformation. Le vecteur (ou plasmide) peut-être un vecteur navette entre *E. coli* et un autre microorganisme.

Le choix de la souche optimisée pour le ratio NADPH/NADP⁺ sera déterminé en fonction du type de biotransformation (fermentation ou bioconversion), de la demande totale en NADPH de la voie de bioconversion considérée, de la nature de(s) source(s) carbonée(s), de la demande en flux de biomasse; ...

La délétion des gènes codant pour les activités Phosphoglucose isomérase et/ou Phosphofructokinase devrait s'imposer lorsque l'on n'est pas capable de maîtriser la répartition du flux de carbone entre la glycolyse et la voie des pentoses phosphate. La délétion des gènes codant pour la Phosphoglucose isomérase sera préférentiellement retenue pour les fermentations ou lorsque la demande en NADPH nécessite, au minimum, un flux de réduction de 2 moles de NADP⁺ par mole de glucose importée. La délétion des gènes codant pour la Phosphofructokinase sera préférentiellement choisie pour les bioconversions ou lorsque la demande en NADPH nécessite, au minimum, un flux de réduction de 3-4 moles de NADP⁺ par mole de glucose importée. La modification, telle que décrite ci-dessus et ci-après, des gènes codant pour les activités Dihydrolipoamide déshydrogénase et/ou Glycéraldéhyde 3-phosphate déshydrogénase sera réalisée lorsque les biotransformations nécessiteront un flux de réduction, au minimum, supérieur à 3 moles de NADP⁺ par mole de glucose importé et notamment, pour optimiser les souches *E. coli* Δ*(udh*A*, qor)* ou *E. coli* Δ*(udh*A*, qor, pgi)* ou *E. coli* Δ*(udh*A*, qor, pfk*A*, pfkB).* Les autres modifications citées, à savoir la surexpression d'au moins un gène codant pour une activité enzymatique parmi la Glucose 6-phosphate déshydrogénase, la 6-Phosphogluconolactonase, la 6-Phosphogluconate déshydrogénase, l'Isocitrate déshydrogénase et la Transhydrogénase membranaire, et/ou la délétion d'au moins un gène codant pour une activité enzymatique parmi la 6-Phosphogluconate déshydratase, la Malate synthase, l'Isocitrate lyase et l'Isocitrate déshydrogénase kinase/phosphatase, pourront être réalisées afin d'affiner l'optimisation du ratio NADPH/NADP⁺ aux besoins de la cellule et du procédé de biotransformation considéré.

La présente invention concerne également un procédé de préparation des souches optimisées selon l'invention telle que définie ci-dessus et ci-après, dans lequel on délète un gène pris parmi ceux codant pour les activités Quinone oxidoréductase et Transhydrogénase soluble, et le cas échéant on délète un gène pris parmi ceux codant pour les activités Glucose 6-phosphate déshydrogénase et 5-Phosphogluconolactonase, et/ou on modifie au moins un gène codant des enzymes à NAD, en particulier pour les activités Dihydrolipoamide déshydrogénase et Glycéraldéhyde 3-phosphate déshydrogénase, afin qu'elles utilisent préférentiellement le NADP, et le cas échéant on délète au moins un gène choisi parmi ceux codant pour les activités 6-Phosphogluconate déshydratase, Malate synthase, Isocitrate lyase et Isocitrate déshydrogénase kinase/phosphatase, ces délétions et modifications étant réalisées par un moyen approprié, et/ou on surexprime au moins un gène choisi parmi ceux codant pour les activités Glucose 6-phosphate déshydrogénase, 6-Phosphogluconolactonase, 6-Phosphogluconate déshydrogénase, Isocitrate déshydrogénase et Transhydrogénase membranaire, soit en transformant la souche avec un vecteur approprié permettant la surexpression, soit en modifiant la force du promoteur endogène contrôlant le gène à surexprimer.

Selon un mode particulier de réalisation de l'invention, le procédé de préparation des souches selon l'invention comprend également la transformation des souches optimisées avec au moins un vecteur approprié comprenant un ou plusieurs gène(s) codant une ou des enzyme(s) impliqués dans la biotransformation d'une molécule d'intérêt, ainsi qu'un ou plusieurs gène(s) marqueur(s) de sélection.

Un autre aspect de l'invention concerne l'utilisation de ces souches optimisées selon l'invention pour les biotransformations NADPH-dépendantes permettant ainsi une amélioration du rendement de biotransformation par rapport à une souche non optimisée pour le NADPH.

Les biotransformations seront réalisées en utilisant des souches définies selon l'invention dans lesquelles seront exprimés des gènes codants pour des activités enzymatiques catalysant des réactions NADPH-dépendantes. L'homme du métier saura aisément identifier de telles enzymes, on citera pour exemples et sans que cette liste soit limitative les enzymes suivantes : EC 1.1.1.10 L-xylulose réductase, EC 1.1.1.21 méthylglyoxal réductase, EC 1.1.1.51 3(or 17)β-hydroxystéroide déshydrogénase, EC 1.1.1.54 allyl-alcohol déshydrogenase, EC 1.1.1.80 isopropanol déshydrogénase, EC 1.1.1.134 dTDP-6-déoxy-L-talose 4-déshydrogénase, EC 1.1.1.149 20α-hydroxystéroide déshydrogénase, EC 1.1.1.151 21-hydroxystéroide déshydrogénase, EC 1.1.1.189 prostaglandine-E₂ 9-réductase, EC 1.1.1.191 indole-3-acétaldehyde réductase EC 1.1.1.207 (-)-menthol déshydrogénase, EC 1.1.1.234 flavanone 4-réductase, EC 1.2.1.50 long-chain-fatty-acyl-CoA réductase, EC 1.3.1.4 cortisone α-réductase, EC 1.3.1.23 cholesténone 5β-réductase, EC 1.3.1.70 Δ¹⁴-stérol réductase, EC 1.4.1.12 2,4-diaminopentanoate déshydrogénase, EC 1.5.1.10 saccharopine déshydrogénase, L-glutamate-forming, EC 1.7.1.6 azobenzène réductase, EC 1.8.1.5 2-oxopropyl-CoM réductase (carboxylating), EC 1.10.1.1 *trans*-acénaphthène-1,2-diol déshydrogénase, EC 1.14.13.7 phenol 2-monooxygénase, EC 1.14.13.12 benzoate 4-monooxygénase, EC 1.14.13.26 phosphatidylcholine 12-monooxygénase, EC 1.14.13.64 4-hydroxybenzoate 1-hydroxylase, EC 1.14.13.70 stérol 14-déméthylase, EC 1.16.1.5 aquacobalamine réductase, EC 1.17.1.1 CDP-4-déhydro-6-déoxyglucose réductase, EC 1.18.1.2 ferredoxine-NADP réductase.

L'invention concerne aussi un procédé de production d'une molécule d'intérêt dont au moins une des réactions de la voie de biosynthèse est NADPH-dépendante, caractérisé en ce qu'il comprend les étapes suivantes :
a) mise en culture des microorganismes optimisés selon l'invention dans un milieu de culture approprié favorisant leur croissance et comprenant les substances nécessaires pour la réalisation de la biotransformation par fermentation ou biotransformation, à l'exception du NADPH, et
b) extraction de la molécule d'intérêt du milieu et le cas échéant purification.

De manière préférentielle, la molécule d'intérêt est choisie parmi les acides aminés, les vitamines, les stérols, les flavonoïdes, les acides gras, les acides organiques, les polyols, les hydroxyesters. Pour les acides aminés ou leurs précurseurs on citera en particulier la lysine, la méthionine, la thréonine, la proline, l'acide glutamique, l'homosérine, l'isoleucine, la valine. Pour les vitamines ou leurs précurseurs on citera notamment le pantoate, le transneurosporène, la phylloquinone, les tocophérols. Pour les stérols on citera notamment le squalène, le cholestérol, la testostérone, la progestérone, la cortisone. Pour les flavonoïdes on citera notamment la frambinone et la vestitone. Pour les acides organiques on citera l'acide coumarique, l'acide 3-hydroxypropionique. Pour les polyols on citera le sorbitol, le xylitol, le glycérol. Pour les hydroxyesters on citera l'éthyl-3-hydroxybutyrate, l'éthyl-4-chloro-3-hydroxybutyrate.

Dans le cas d'une bioconversion, le procédé comprend aussi l'ajout du substrat à « convertir » dans le milieu de culture approprié.

Le milieu de culture cité à l'étape b) du procédé selon l'invention définie ci-dessus comprend au moins un carbohydrate assimilable choisi parmi différents sucres assimilables, tels le glucose, le galactose, le saccharose, le lactose, ou les mélasses, ou les sous-produits de ces sucres. Une source de carbone simple tout particulièrement préférée est le glucose. Une autre source de carbone simple préférée est le saccharose. Le milieu de culture peut en outre contenir une ou plusieurs substances (e.g. acides aminés, vitamines, sels minéraux, ...) favorisant la croissance du microorganisme et/ou la production de la molécule d'intérêt. En particulier, le milieu minéral de culture pour *E. coli* pourra ainsi être de composition identique ou similaire à un milieu M9 (Anderson, 1946, Proc. Natl. Acad. Sci. USA 32:120-128), un milieu M63 (Miller, 1992 ; A Short Course in Bacterial Genetics: A Laboratory Manual and Handbook for Escherichia coli and Related Bacteria, Cold Spring Harbor Laboratory Press, Cold Spring Harbor, New York) ou un milieu tel que défini par Schaefer et al. (1999, Anal. Biochem. 270 : 88-96).

La définition des conditions de biotransformation est du ressort de l'homme du métier. On fermente notamment les microorganismes à une température comprise entre 20°C et 55°C, de préférence entre 25°C et 40°C, plus particulièrement d'environ 30°C pour *S. cerevisiae* et d'environ 37°C pour *E. coli.*

Les exemples ne sont donnés qu'à titre d'illustration de l'invention et ne limitent en aucun le mode de réalisation ni la portée de l'invention.

### Exemple 1 : Calcul des rendements optimaux théoriques de bioconversion de l'éthylacétoacétate en éthyl-3-hydroxybutyrate

### a) bioconversion avec E. coli

Des modélisations prédictives sont réalisées en utilisant l'algorithme MetOpt®-Coli, un modèle stoechiométrique développé par la société METabolic EXplorer, qui permet de définir 1) le rendement maximal de production en éthyl-3-hydroxybutyrate à partir de l'éthylacétoacétate 2) la meilleure distribution de flux à partir du glucose pour assurer les besoins de croissance et d'équilibres redox nécessaires pour le développement de la cellule et l'atteinte du rendement maximal de bioconversion.

Les paramètres imposés au modèle sont notamment 1) un flux d'import de glucose à 3 mmol.g⁻¹.h⁻¹, 2) un taux de croissance variable de 0, 0.15 et 0.25 h⁻¹, 3) un flux de la transhydrogénase membranaire (*pnt*AB) variable et inférieur ou égal à 1 mmol.g⁻¹.h⁻¹. La valeur limite de flux de la transhydrogénase membranaire est déterminée à partir de la littérature (Hanson, 1979 ; *Anderlund et al.,* 1999 ; Emmerling *et al.,* 2002) ; 4) le flux de maintenance a été limité entre 5 et 22 mmol.g⁻¹.h⁻¹.

Dans tous les cas le modèle suggère la délétion des gènes *udh*A et *qor.* En pratique toutefois, la souche E. coli [Δ(*udh*A, *qor*)] ne permettra pas d'obtenir un rendement équivalent au rendement optimum théorique, car il sera difficile de maintenir la répartition adéquate de flux de carbone entre la voie des pentoses phosphate et celle de la glycolyse, cette répartition étant variable avec le taux de croissance. En pratique, on préférera donc utiliser les souches *E. coli* [Δ(*udh*A, *qor*, *pfk*A, *pfk*B)] ou [Δ(*udh*A, *qor*, *pgi*)]*,* le choix entre ces deux souches étant fonction du taux de croissance de la souche lors du procédé de bioconversion.

Les rendements optimaux théoriques de bioconversion de l'éthylacétoacétate en éthyl-3-hydroxybutyrate ont été calculés pour différentes souches de *E. coli* optimisées selon l'invention :

| | **µ = 0** | **µ=0,15 h⁻¹** | **N=0,25h⁻¹** |
|---|---|---|---|
| Δ(*udhA, qor, pgi*) | 1,82 | 1,74 | 1,22 |
| Δ(*udhA, qor, pgi*) gapA-NADP dépendant | 4,29 | 3,64 | 2,43 |
| Δ(*udhA, qor, pgi*) *lpd-*NADP dépendant | 5,67 | 3,46 | 1,99 |
| Δ(*udhA, qor, pgi*) *gap*A-NADP dépendant *lpd*-NADP dépendant | 6,86 | 4,96 | 3,33 |
| Δ(*udhA, qor, pfk*A*, pfkB*) | 6,76 | 4,65 | 0,19 |
| Δ(*udhA, qor, pfk*A*, pfkB*) *gap*A-NADP dépendant | 8,16 | 5,54 | 1,02 |
| Δ(u*dhA, qor, pfk*A*, pfkB*) *lpd*-NADP dépendant | 8,33 | 5,60 | 1,77 |
| Δ(*udhA, qor, pfk*A*, pfkB*) gapA-NADP dépendant *lpd-*NADP dépendant | 9,33 | 6,38 | 2,60 |

### Rendements optimaux théoriques de bioconversion de l'éthylacétoacétate en éthyl-3-hydroxybutyrate (mol par mol de glucose) par des souches d'E. coli optimisées dans leur capacité de réduction du NADP⁺

Afin d'améliorer encore le rendement optimal théorique des souches optimisées selon l'invention, des modifications supplémentaires peuvent être prises en compte, telles que la surexpression d'au moins un gène choisi parmi *zwf, gnd, pntA, pntB* et *icd* et/ou la délétion d'au moins un gène choisi parmi *edd, ace*A*, ace*B et *ace*K*.*

### b) bioconversion avec S. cerevisiae

Les modélisations prédictives sont réalisées en utilisant l'algorithme MetOpt®-Scere, un modèle stoechiométrique développé par la société, qui permet de définir 1) le rendement maximal de production en éthyl-3-hydroxybutyrate à partir de l'éthylacétoacétate 2) la meilleure distribution de flux à partir du glucose pour assurer les besoins de croissance et d'équilibres redox nécessaire pour le développement de la cellule et l'atteinte du rendement maximal de bioconversion.

Les paramètres imposés au modèle sont notamment 1) un flux d'import de glucose à 3 mmol.g⁻¹.h⁻¹, 2) un taux de croissance variable de 0, 0.15 et 0.25 h⁻¹, 3) un flux de maintenance inférieur ou égal à 22 mmol.g⁻¹.h⁻¹; 4) les réactions des aldéhydes déshydrogénases (ALD2, ALD3, ALD6) sont irréversibles et imposées dans le sens acétate + NAD(P)H → acétaldehyde + NAD(P) ; 5) la levure ne possède pas d'activités équivalentes à *udh*A ou *pnt*A,B.

Le modèle permet de prendre en compte la compartimentation mitochondriale et peroxisomale.

Dans tous les cas, le modèle suggère la délétion d'un gène codant pour une enzyme oxydant le NADPH et en particulier, du gène ZTA1. En pratique toutefois, la souche S. *cerevisiae* [AZTA1] ne permettra pas d'obtenir un rendement équivalent au rendement optimum théorique, car il sera difficile de maintenir la répartition adéquate de flux de carbone entre la voie des pentoses phosphate et celle de la glycolyse, cette répartition étant variable avec le taux de croissance. En pratique, on préférera donc utiliser les souches *S. cerevisiae* [Δ(ZTA1, PFK1, PFK2)] ou [Δ(ZTA1, PGI1)], le choix entre ces deux souches étant fonction du taux de croissance de la souche lors du procédé de bioconversion.

Les rendements optimaux théoriques de bioconversion de l'éthylacétoacétate en éthyl-3-hydroxybutyrate ont été calculés pour différentes souches de *S. cerevisiae* optimisées selon l'invention :

| | **µ = 0** | **µ=0,15h⁻¹** | **µ=0,25 h⁻¹** |
|---|---|---|---|
| Δ(ZTA1,PGI1) | 2,42 | 2,00 | 1,73 |
| Δ(ZTA1,PGI1) TDH1,2,3-NADP dépendant | 4,22 | 3,50 | 3,03 |
| Δ(ZTA1,PGI1) LPD1-NADP dépendant | 4,08 | 3,29 | 2,77 |
| Δ(ZTA1,PGI1) TDH1,2,3-NADP dépendant LPD1-NADP dépendant | 6,17 | 5,01 | 4,23 |
| Δ(ZTA1, PFK1, PFK2) | 12,00 | 8,18 | 5,64 |
| Δ(ZTA1, PFK1, PFK2) TDH1,2,3-NADP-dépendant | 12,00 | 9,11 | 7,19 |
| Δ(ZTA1, PFK1, PFK2) LPD1-NADP dépendant | 12,00 | 8,44 | 6,06 |
| Δ(ZTA1, PFK1, PFK2) TDH1,2,3-NADP dépendant LPD1-NADP dépendant | 12,00 | 9,28 | 7,46 |

### Rendements optimaux théoriques de bioconversion de l'éthylacétoacétate en éthyl-3-hydroxybutyrate (mol par mol de glucose) par des souches de S. cerevisiae optimisées dans leur capacité de réduction du NADP⁺

Afin d'améliorer encore le rendement optimal théorique des souches optimisées selon l'invention, des modifications supplémentaires peuvent être prises en compte, telles que la surexpression d'au moins un gène choisi parmi ZWF, SOL1, SOL2, SOL3, SOL4, GND1, GND2, IDP1, IDP2 et IDP3 et/ou la délétion d'au moins un gène choisi parmi ICL1, DAL7.

### Exemple 2 : Construction de la souche E. coli [Δ(udhA, qor)]

L'inactivation du gène *udhA* est réalisée par recombinasion homologue d'après la technique décrite par Datsenko et Wanner (One-step inactivation of chromosomal genes in Escherichia coli K-12 using PCR products, Proc. Natl. Acad. Sci. USA,2000, 97 : 6640-6645).

Cette technique consiste à insérer une cassette de résistance à un antibiotique (chloramphénicol) tout en délétant la majeure partie du gène concerné. Pour cela on synthétise une paire d'oligonucléotides, chacun étant constitués de 100pb dont 80pb (minuscules) sont homologues avec le gène à déléter (e.g. *udhA)* et 20pb (majuscules) sont homologues avec la cassette de résistance au chloramphénicol :
DudhAF
DudhAR

La cassette antibiotique portée par le plasmide pKD3 est amplifiée par PCR en utilisant les oligonucléotides DudhAF et DudhAR. Le produit PCR obtenu est alors introduit par électroporation dans la souche *E. coli* [pKD46], qui porte le gène codant la Red recombinase; enzyme catalysant la recombinaison homologue. Les transformants résistants au chloramphénicol sont alors sélectionnés et l'insertion de la cassette de résistance est vérifiée par une analyse PCR en utilisant les oligonucléotides UdhAF et UdhAR :
UdhaF
   Ggccgctcaggatatagccagataaatgac
UdhaR
   Gcgggatcactttactgccagcgctggctg

La cassette de résistance au chloramphénicol est ensuite éliminée. Pour cela, le plasmide pCP20 porteur de la recombinase FLP agissant au niveau des sites FRT de la cassette de résistance au chloramphénicol, est introduit dans les souches recombinantes par électroporation. Puis, après une série de cultures à 42°C, la perte de la cassette de résistance à l'antibiotique est vérifiée par une analyse PCR avec les oligonucléotides UdhAF et UdhAR.

L'inactivation du gène *qor* est réalisée selon la même technique en utilisant les oligonucléotides suivants :
DqorF
DqorR
QorF
   Cgcccaacaccgactgctccgcttcgatcg
QorR
   cagcgttatgaccgctggcgttactaaggg

Pour des raisons pratiques, il peut être intéressant de déléter les deux gènes simultanément. Pour cela chaque gène est remplacé par une cassette de résistance à un antibiotique différent (par exemple chloramphénicol pour *udhA* et kanamycine pour *qor).*

La souche obtenue est donc *E. coli* [Δ(*udh*A, *qor*)]*.*

### Exemple 3 : Construction du plasmide pSK-PgapA-GRE2p, introduction dans la souche E. coli [Δ(udhA, qor)] et biotransformation de l'éthylacétoacétate en éthyl-3-hydroxybutyrate

Le plasmide pSK-PgapA est construit par insertion du promoteur *gapA* dans le vecteur pBluescript-SK (pSK). Pour cela, le promoteur *gapA d'E. coli* est amplifié avec la polymerase *Pwo* à partir de l'ADN chromosomique.

Le produit PCR obtenu est ensuite digéré par l'enzyme de restriction Hin*d*III et ligaturé au vecteur pSK digéré par l'enzyme de restriction *Hind*III et déphosphorylé pour donner le plasmide pSK-PgapA. Le vecteur pSK porte une origine de réplication pour *E. coli* et un gène de résistance à l'ampicilline.

Le plasmide pSK-PgapA est alors introduit dans la souche *E. coli* DH5α pour vérification de la construction. Le séquençage du promoteur *gapA* du plasmide pSK-PgapA avec l'oligonucléotide universel M13 forward est ensuite réalisé pour confirmer la construction.

Le plasmide pSK-PgapA-GRE2p est construit par insertion du gène GRE2p dans le plasmide pSK-PgapA. Pour cela, le gène GRE2p de *Saccharomyces cerevisiae* est amplifié avec la polymerase *Pwo* à partir de l'ADN chromosomique en utilisant les oligonucléotides suivants :
Ome 119_GRE2F (Ndel)
   Acgtacgtggcatatgtcagttttcgtttcaggtgctaacggg
Ome120_GRE2R (PstI)
   Acgtacctgcagttatattctgccctcaaattttaaaatttggg

Le produit PCR obtenu est ensuite digéré par les enzymes de restriction *NdeI - PstI* et ligaturé au vecteur pSK-PgapA digéré par les enzymes de restriction *NdeI - PstI* et déphosphorylé pour donner le plasmide pSK-PgapA-GRE2p. Le plasmide pSK-PgapA porte une origine de réplication pour *E. coli* et un gène de résistance à l'ampicilline.

Le plasmide pSK-PgapA-GRE2p est alors introduit dans la souche *E. coli* DH5α pour vérification de la construction. Le séquençage du gène GRE2p du plasmide pSK-PgapA-GRE2p avec les oligonucléotides universels M13 reverse et M13 forward est ensuite réalisé pour confirmer la construction.

Le plasmide validé est alors introduit dans la souche *E. coli* [Δ(*udh*A, *qor*)] (exemple 2) par électroporation.

La souche obtenue *E. coli* [Δ(*udh*A, *qor*) pSK-PgapA-GRE2p] est alors cultivée en milieu minimum contenant du glucose et de l'éthylacétoacétate. Une souche d'*E. coli* [pSK-PgapA-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *E. coli* [Δ(*udh*A*, qor*) pSK-PgapA-GRE2p] présente un rendement de production d'éthyl-3-hydroxybutyrate accru par rapport à la souche non optimisée.

### Exemple 4 : Construction de la souche E. coli [Δ(udhA,qor,pgi) pSK-PgapA-GRE2p] et biotransformation de l'éthylacétoacétate en éthyl-3-hydroxybutvrate

L'inactivation du gène *pgi* est conduite dans la souche *E. coli* [Δ(*udh*A*, qor*)] (exemple 2) en utilisant la technique décrite dans l'exemple 2, et les oligonucléotides suivants :
DpgiF
DpgiR
pgiF
   gcggggcggttgtcaacgatggggtcatgc
pgiR
   cggtatgatttccgttaaattacagacaag

La construction est réalisée en milieu riche (*e.g*. LB). On introduit alors dans la souches obtenue le plasmide pSK-PgapA-GRE2p (exemple 3) par électroporation, et la souche résultante *E. coli* [Δ(*udh*A*, qor, pgi*) pSK-PgapA-GRE2p] est sélectionnée sur milieu riche.

La souche obtenue est alors cultivée en milieu minimum contenant du glucose et de l'éthylacétoacétate. Une souche d'*E. coli* [pSK-PgapA-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *E. coli* [Δ(*udh*A*, qor, pgi*) pSK-PgapA-GRE2p] présente un rendement de production d'éthyl-3-hydroxybutyrate accru par rapport à la souche non optimisée.

| | mol_{EHB}/mol_{Glucose} |
|---|---|
| MG1655 pSK-PgapA-GRE2p | 0,75 |
| *MG1655* Δ(*udhA, qor, pgi*) pSK-PgapA-GRE2p | 2,12 |

### Exemple 5 : Construction de la souche E. coli [Δ(udhA, qor, pgi, edd) pSK-PgapA-GRE2p] et biotransformation de l'éthylacétoacétate en éthyl-3-hydroxybutyrate

L'inactivation du gène *edd* est conduite dans la souche *E*. *coli* [Δ(*udh*A*, qor, pgi*)] (exemple 4) en utilisant la technique décrite dans l'exemple 2, et les oligonucléotides suivants :
DeddF (1932582-1932499)
DeddR (1930866-1930943)
EddF (1932996-1932968)
   Gggtagactccattactgaggcgtgggcg
EddR (1930439-1930462)
   Ccccggaatcagaggaatagtccc

La construction est réalisée en milieu riche (*e.g*. LB). On introduit alors dans la souche obtenue *E. coli* [Δ(udh*A, qor, pgi, edd*)] le plasmide pSK-PgapA-GRE2p (exemple 3) par électroporation, et la souche résultante *E. coli* [Δ(*udhA, qor, pgi, edd*) pSK-PgapA-GRE2p] est sélectionnée sur milieu riche.

La souche obtenue *E. coli* [A(*udhA, qor, pgi, edd*) pSK-PgapA-GRE2p] est alors cultivée en milieu minimum contenant du glucose et de l'éthylacétoacétate. Une souche *E. coli* [pSK-PgapA-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *E. coli* [Δ(*udhA, qor, pgi, edd*) pSK-PgapA-GRE2p] présente un rendement de production d'éthyl-3-hydroxybutyrate accru par rapport à la souche non optimisée.

### Exemple 6 : Construction de la souche E. coli [Δ(udhA, gor, pfkA, pfkB) pSK-PgapA-GRE2p] et biotransformation de l'éthylacétoacétate en éthyl-3-hydroxvbutyrate

L'inactivation des gènes *pfk*A et *pfkB* est conduite dans la souche *E. coli* [Δ(udhA*, qor*)] (exemple 2) en utilisant la technique décrite dans l'exemple 2, et les oligonucléotides suivants :
DpfkAF DpfkAR
PtkAF
   Cgcacgcggcagtcagggccgacccgc
PfkAR
   ccctacgccccacttgttcatcgcccg
DpfkBF (1804421-1804499)
DpfkBR (1805320-1805241)
PfkBF (1803996-1804025)
   tggcaggatcatccatgacagtaaaaacgg
PfkBR (1805657-1805632)
   gccggttgcactttgggtaagccccg

La construction est réalisée en milieu riche (*e.g*. LB). On introduit alors dans la souche obtenue *E. coli* [Δ(*udh*A*, qor, pfkA, pfkB*)] le plasmide pSK-PgapA-GRE2p (exemple 3) par électroporation, et la souche résultante *E*. *coli* [Δ(*udh*A*, qor, pfkA, pfkB*) pSK-PgapA-GRE2p] est sélectionnée sur milieu riche.

La souche obtenue *E*. *coli* [Δ(*udh*A*, qor, pfkA, pfkB)* pSK-PgapA-GRE2p] est alors cultivée en milieu minimum contenant du glucose et de l'éthylacétoacétate. Une souche *E. coli* [pSK-PgapA-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *E. coli* [Δ(*udh*A, *qor, pfkA, pfkB*) pSK-PgapA-GRE2p] présente un rendement de production d'éthyl-3-hydroxybutyrate accru par rapport à la souche non optimisée.

| | mol_{EHB}/mol_{Glucose} |
|---|---|
| MG1655 pSK-PgapA-GRE2p | 0,75 |
| *MG1655* Δ(*udhA, qor, pfkA, pfkB)* pSK-PgapA-GRE2p | 3,46 |

### Exemple 7 : Construction de la souche E. coli [Δ(udhA, qor, pgi, lpd) plpd*, pSK-PgapA-GRE2p] et biotransformation de l'éthylacétoacétate en éthyl-3-hvdroxybutyrate

Le gène *lpd* codant la dihydrolipoamide déshydrogénase NAD-dépendante, impliquée dans le complexe multienzymatique pyruvate déshydrogénase, est délété en utilisant la technique décrite dans l'exemple 2 sauf que la souche initiale est la souche *E. coli* [Δ(*udh*A*, qor, pgi*)] décrite dans l'exemple 4 au lieu d'être une souche sauvage. La construction et la sélection de la souche modifiée est réalisée en milieu riche (*e.g*. LB). La souche obtenue est *E.coli* [Δ(*udh*A*, qor, pgi, lpd*)]*.*

Par ailleurs on construit le plasmide p*-lpd** qui permet la surexpression d'une dihydrolipoamide déshydrogénase NADP-dépendante. Il existe différentes possibilités pour modifier la spécificité de cofacteur d'une enzyme. Par exemple Bocanegra *et al.* (1993) divulguent une méthode pour créer une dihydrolipoamide déshydrogénase NADP-dépendante.

Les plasmides *p-lpd** et pSK-PgapA-GRE2p sont alors introduit par électroporation dans la souche *E.coli* [Δ(*udhA, qor, pgi, lpd*)]*,* alternativement, on peut choisir de cloner *lpd** sur pSK-PgapA-GRE2p ; on obtiendrait alors le plasmide pSK-PgapA-GRE2p-lpd* que l'on introduirait par éléctroporation dans la souche *E.coli* [Δ(*udhA, qor, pgi, lpd*)]*.* La construction et la sélection de la souche modifiée est réalisée en milieu riche (*e.g*. LB).

La souche obtenue *E. coli* [Δ(*udhA, qor, pgi, lpd*) pSK-PgapA-GRE2p, *p-lpd**)] est alors cultivée en milieu minimum contenant du glucose et de l'éthylacétoacétate. Une souche *E. coli* [pSK-PgapA-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *E. coli* [Δ(*udhA, qor, pgi, lpd*) pSK-PgapA-GRE2p, p*-lpd**)] présente un rendement de production d'éthyl-3-hydroxybutyrate accru par rapport à la souche non optimisée.

### Exemple 8 : Construction du plasmide pRSGK-GRE2p

Le plasmide pYGK est construit par insertion du promoteur *Ppgk,* du site de multiclonage et du terminateur *cyc1* du vecteur pYPG2 dans le vecteur pBluescript-SK (pSK). Pour cela, le promoteur P*pgk,* le site de multiclonage et le terminateur *cyc1* sont amplifiés avec la polymerase *Pfu Turbo* à partir du vecteur pYPG2. Le produit PCR obtenu est ensuite digéré par les enzymes de restriction *SacII - NotI* et ligaturé au vecteur pSK digéré par les enzymes de restriction *tapaI - SmaI,* ligaturé puis digéré par les enzymes de restriction NotI - SacII et déphosphorylé pour donner le plasmide pYGK.

Le plasmide pYGK est alors introduit dans la souche E. coli DH5α pour vérification de la construction. Le séquençage du promoteur P*pgk*, du site de multiclonage et du terminateur *cyc1* du plasmide pYGK avec les oligonucléotides universels M13 reverse et M13 forward est ensuite réalisé pour confirmer la construction.

Le plasmide pYGK-GRE2p est ensuite construit par insertion du gène GRE2p dans le plasmide pYGK. Pour cela, le gène GRE2p de Saccharomyces cerevisiae est amplifié avec la polymerase *Pwo* à partir de l'ADN chromosomique, en utilisant les oligonucléotides suivants :
Ome376_Gre2 pYGK F (SmaI)
   Acgtacgtc**ccccgg**aaaaatgtcagttttcgtttcaggtgc
Ome377_Gre2 pYGK R (ApaI)
   ACGTAC**GGGCCC**TTATATTCTGCCCTCAAATTTTAAAATTTGGG

Le produit PCR obtenu est ensuite digéré par les enzymes de restriction *ApaI - SmaI* et ligaturé au vecteur pYGK digéré par les enzymes de restriction *ApaI - SmaI* et déphosphorylé pour donner le plasmide pYGK-GRE2p.

Le plasmide pYGK-GRE2p est alors introduit dans la souche *E. coli* DH5α pour vérification de la construction. Le séquençage du gène GRE2p du plasmide pYGK-GRE2p avec les oligonucléotides universels M13 reverse et M13 forward est ensuite réalisé pour confirmer la construction.

Le plasmide pRSGK-GRE2p est finalement obtenu par digestion des plasmides pYGK-GRE2p et pRS426 avec les enzymes de restriction *NotI - SacII* puis ligation.

### Exemple 9 : Construction de la souche S. cerevisiae [Δ(ZTA1) pRSGK-GRE2p] et biotransformation de l'éthylacétoacétate en éthyl-3-hydroxybutyrate

L'inactivation du gène ZTA1 est réalisée en insérant un marqueur (résistance à un antibiotique, auxotrophie) tout en délétant la majeure partie du gène concerné. La technique utilisée est décrite par Brachmann et al. (Designer deletion strains derived from Saccharomyces cerevisae S288C : a useful set of strains and plasmids for PCR-mediated gene disruption and other applications, Yeast, 1998, 14: 115-32). On peut aussi utiliser la technique décrite par Wach et al. (New heterologous modules for classical or PCR-based gene disruptions in Saccharomyces cerevisiae, Yeast, 1994, 10 : 1793-1808).

Dans tous les cas on obtient une souche finale *S. cerevisiae* [Δ(ZTA1)], dans laquelle le plasmide pRSGK-GRE2p (exemple 8) est alors introduit.

Alternativement, on peut aussi choisir d'introduire le plasmide pRSGK-GRE2p dans une souche Δ(ZTA1) disponible, par exemple la souche EUROSCARF Y33183 (génotype : BY4743; Mat a/α; *his*3D1/*his*3D1; leu2D0/leu2D0; *lys*2D0/*LYS2*; *MET*15/*met15*D0; *ura3*D0/*ura3*D0; *YBR046c::kan*MX4/*YBR046c::kan*MX4). II est alors possible après sporulation de récupérer une souche homozygote *S. cerevisiae* [Δ(ZTA1) pRSGK-GRE2p].

La souche obtenue *S. cerevisiae* [Δ(ZTA1) pRSGK-GRE2p] est ensuite cultivée en milieu minimum contenant du glucose et de l'éthylacétoacétate.

La souche témoin *S. cerevisiae* [pRSGK-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *S*. *cerevisiae* [Δ(ZTA1) pRSGK-GRE2p] présente un rendement de production d'éthyl-3-hydroxybutyrate accru par rapport à la souche non optimisée.

### Exemple 10 : Construction de la souche S. cerevisiae [Δ(ZTA1, PGI1) pRSGK-GRE2p] et biotransformation de l'éthylacétoacétate en éthyl-3-hydroxybutyrate

L'inactivation du gène PGI1 est réalisée dans la souche *S*. *cerevisiae* [Δ(ZTA1) pRSGK-GRE2p] en utilisant la technique décrite dans l'exemple 9, et les oligonucléotides suivants :
Dpgi1F
DpgilR
PgilF
   GCGGGGCGGTTGTCAACGATGGGGTCATGC
Pgi1R
   CGGTATGATTTCCGTTAAATTACAGACAAG

Alternativement il est possible d'utiliser une souche A(PGI1) disponible, par exemple la souche EUROSCARF Y23336 (Mat α/a ; his 3D1/his3D1; leu2D0/leu2D0; lys2D0/LYS2; MET15/met15D0; ura3D0/ura3D0; YBR196c::kanMX4/YBR196c). La souche est alors transformée par le plasmide pRSGK-GRE2p (exemple 8) puis la délétion du gène ZTA1 est réalisée en utilisant la technique décrite dans l'exemple 9.

La souche obtenue *S*. *cerevisiae* [Δ(ZTA1, PGI1) pRSGK-GRE2p] est alors cultivée en milieu minimum contenant du glucose et de l'éthylacétoacétate.

La souche témoin *S*. *cerevisiae* [pRSGK-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *S*. *cerevisiae* [Δ(ZTA1, PGI1) pRSGK-GRE2p] présente un rendement de production d'éthyl-3-hydroxybutyrate accru par rapport à la souche non optimisée.

### Exemple 11 : Construction de la souche S. cerevisiae [Δ(ZTA1, PFK1, PFK2) pRSGK-GRE2p] et biotransformation de l'éthylacétoacétate en éthyl-3-hydroxybutyrate

Les gènes PFK1 et PFK2 sont délétés dans la souche *S. cerevisiae* [Δ(ZTA1)] en utilisant la technique décrite dans l'exemple 9, et les oligonucléotides suivants :
Dpfk1F
Dpfk1R
Pfk1 int F
   GCTTTCTTAGAAGCTACCTCCG
Pfk1 int R
   GAACCGACAAGACCAACAATGG
Pfk2 int F
   CAGTTGTACACTTTGGACCC
Pfk₂ int R
   GATCAGCACCAGTCAAAGAACC

La souche est alors transformée par le plasmide pRSGK-GRE2p (exemple 8).

La souche obtenue *S*. *cerevisiae* [Δ(ZTA1, PFK1, PFK2) pRSGK-GRE2p] est alors cultivée en milieu minimum contenant du glucose et de l'éthylacétoacétate.

La souche témoin *S*. *cerevisiae* [pRSGK-GRE2p] est cultivée dans les mêmes conditions.

Lorsque les cultures sont achevées on compare :
- l'évolution de la biomasse de chaque souche pendant la phase de bioconversion,
- la quantité d'éthyl-3-hydroxybutyrate produit dans le milieu extracellulaire,
- la quantité d'éthyl-3-hydroxybutyrate accumulé dans les cellules,
- la productivité en éthyl-3-hydroxybutyrate,
- le rendement glucose/éthyl-3-hydroxybutyrate.

On pourra observer que la souche *S*. *cerevisiae* [Δ(ZTA1, PFK1, PFK2) pRSGK-GRE2p] présente un rendement de production d'éthyl-3-hydroxybutyrate accru par rapport à la souche non optimisée.

| | mol_{EHB}/mol_{Glucose} |
|---|---|
| *S. cerevisiae* [pRSGK-GRE2p] | en cours |
| *S. cerevisiae* [Δ(ZTA1, PFK1, PFK2) pRSGK-GRE2p] | en cours |

### Exemple 12 : Comparaison entres valeurs expérimentales et prédictions par le modèle métabolique pour l'optimisation de la production d'éthyl-3-hydroxybutyrate par Escherichia coli

On pourra observer une bonne corrélation entre les modélisations prédictives (exemple 1) et les réalisations expérimentales décrites dans les exemples 3, 4, 5, 6, 7, 9, 10 et 11.

Les exemples 1 à 11 ci-dessus sont des applications particulières du brevet et n'en limitent pas l'utilisation. L'homme de l'art saura aisément adapter ces exemples pour la biotransformation de molécules ayant une synthèse NADPH-dépendante. L'algorithme MetOpt^{®} et la stratégie d'optimisation d'un procédé de bioconversion NADPH-dépendant via l'optimisation du ratio NADPH/NADP⁺ est validé ; cela nous permet en outre de revendiquer une application élargie à toutes les biotransformations NADPH dépendantes, qui pourront être modélisées et prévues par MetOpt^{®} ou l'un de ses dérivés, en utilisant *E. coli*, *S. cerevisiae* ou tout autre microorganisme.

### Exemple 13 : Calcul des rendements optimaux théoriques dans le cadre de procédés de fermentation chez E. coli

L'exemple 12 montre que les modèles MetOpt® développés par la société sont applicables aux bioconversions et devraient plus généralement s'appliquer aux biotransformations telles que les fermentations.

A titre d'exemples, le modèle MetOpt®-Coli est appliqué à la production de cystéine ou de 3-hydroxypropionate par fermentation du glucose dans des souches d'*E. coli* optimisées selon l'invention. Les paramètres utilisés sont les mêmes que dans l'exemple 1, à savoir : 1) un flux d'import de glucose à 3 mmol.g⁻¹.h⁻¹, 2) un taux de croissance variable de 0, 0.15 et 0.25 h⁻¹, 3) un flux de la transhydrogénase membranaire (*pnt*AB) variable et inférieur ou égal 1 mmol.g⁻¹.h⁻¹. La valeur limite de flux de la transhydrogénase membranaire est déterminée à partir de la littérature (Hanson, 1979 ; Anderlund *et al.*, 1999 ; Emmerling *et al.*, 2002) ; 4) le flux de maintenance a été limité entre 5 et 22 mmol.g⁻¹.h⁻¹.

### a) cas de la production de cystéine par fermentation du glucose

| | **µ=0** | **µ=0,15h⁻¹** | **µ=0,25h⁻¹** |
|---|---|---|---|
| Δ(*udhA*, *qor*, *pgi*) | 0,66 | 0,37 | 0,09 |
| Δ(*udhA*, *qor*, *pgi*) *gap*A-NADP dépendant | 0,78 | 0,37 | 0,09 |
| Δ(*udhA*, *qor*, *pgi*) *lpd*-NADP dépendant | 0,78 | 0,37 | 0,09 |
| *Δ*(*udhA*, *qor*, *pgi*) *gap*A-NADP dépendant *lpd*-NADP dépendant | 0,78 | 0,37 | 0,09 |
| Δ(*udhA*, *qor*, *pfkA*, *pfkB*) | 0,40 | 0,18 | 0,01 |
| Δ(*udhA*, *qor*, *pfkA*, *pfkB*) *gap*A-NADP dépendant | 0,62 | 0,30 | 0,06 |
| Δ(*udhA*, *qor*, *pfkA*, *pfkB*) *Ipd*-NADP dépendant | 0,71 | 0,36 | 0,13 |
| Δ(*udhA*, *qor*, *pfkA*, *pfkB*) *gap*A-NADP dépendant *lpd*-NADP dépendant | 0,77 | 0,42 | 0,17 |

### Rendements optimaux théoriques de production de la cystéine par des souches d'E. coli optimisées dans leur capacité de réduction du NADP⁺ (mol par mol de glucose)

Afin d'améliorer encore le rendement optimal théorique des souches optimisées selon l'invention, des modifications supplémentaires peuvent être prises en compte, telles que la surexpression d'au moins un gène choisi parmi *zwf*, *gnd*, *pnt*A, *pnt*B et *icd* et/ou la délétion d'au moins un gène choisi parmi *edd*, *ace*A, *ace*B et *ace*K.

Dans la pratique pour obtenir de tels rendements, d'autres modifications devront être apportées aux souches optimisées selon l'invention, par exemple en surexprimant le gène *cysB* comme décrit dans le brevet WO0127307, ou en modifiant le gène *cysE* comme décrit dans le brevet EP0885962.

### b) cas de la production de 3-hydroxypropionate par fermentation du glucose

La production de 3-hydroxypropionate est réalisée dans des souches d'*E. coli* contenant les gènes codant pour les enzymes de la voie de synthèse du 3-hydroxypropionate, par exemple la malonyl-coA reductase de *Chloroflexus aurantiacus* (Hügler et al., Journal of Bacteriology, 2002, 184 : 2404-2410).

| | **µ=0** | **µ=0,15h⁻¹** | **µ = 0,25 h⁻¹** |
|---|---|---|---|
| Δ(*udhA*, *qor*, *pgi*) | 1,33 | 0,79 | 0,30 |
| Δ(*udhA*, *qor*, *pgi*) *gap*A-NADP dépendant | 1,76 | 0,99 | 0,30 |
| Δ(*udhA*, *qor*, *pgi*) *lpd*-NADP dépendant | 1,82 | 0,99 | 0,30 |
| Δ(*udhA*, *qor*, *pgi*) *gap*A-NADP dépendant *lpd*-NADP dépendant | 1,82 | 0,99 | 0,30 |
| Δ(*udhA*, *qor*, *pfk*A, *pfkB*) | 1,62 | 0,66 | 0,03 |
| Δ(*udhA*, *qor*, *pfk*A, *pfkB*) *gap*A-NADP dépendant | 1,76 | 0,79 | 0,07 |
| Δ(*udhA*, *qor*, *pfk*A, *pfkB*) *Ipd*-NADP dépendant | 1,79 | 0,84 | 0,07 |
| Δ(*udhA*, *qor*, *pfk*A, *pfkB*) *gap*A-NADP dépendant *lpd*-NADP dépendant | 1,79 | 0,84 | 0,07 |

### Rendements optimaux théoriques de production de 3-hydroxypropionate par des souches d'E. coli optimisées dans leur capacité de réduction du NADP⁺ (mol par mol de glucose)

Afin d'améliorer encore le rendement optimal théorique des souches optimisées selon l'invention, des modifications supplémentaires peuvent être prises en compte, telles que la surexpression d'au moins un gène choisi parmi *zwf*, *gnd*, *pnt*A, *pnt*B et *icd* et/ou la délétion d'au moins un gène choisi parmi *edd, ace*A, *ace*B et *ace*K.

### Exemple 14 : Calcul des rendements optimaux théoriques dans le cadre de procédés de fermentation chez S. cenevisiae ; application à la production d'hydrocortisone

L'exemple 12 montre que les modèles MetOpt® développés par la société sont applicables aux bioconversions et devraient plus généralement s'appliquer aux biotransformations telles que les fermentations.

A titre d'exemple, le modèle MetOpt®-Scere est appliqué à la production d'hydrocortisone par fermentation du glucose dans des souches de *S*. *cerevisiae* optimisées selon l'invention. Les paramètres utilisés sont les mêmes que dans l'exemple 1, à savoir : 1) un flux d'import de glucose à 3 mmol.g⁻¹.h⁻¹, 2) un taux de croissance variable de 0, 0.15 et 0.25 h⁻¹, 3) un flux de maintenance inférieur ou égal à 22 mmol.g⁻¹.h⁻¹ ; 4) les réactions des aldéhydes déhydrogénases (ALD2, ALD3, ALD6) sont irréversibles et imposées dans le sens acétate + NAD(P)H → acétaldehyde + NAD(P) ; 5) la levure ne possède pas d'activités équivalentes à *udh*A ou *pnt*A,B.

Le modèle permet de prendre en compte la compartimentation mitochondriale et péroxisomale.

Cette représentation des résultats permet de mettre en évidence l'apport réel de chacune des mutations apportées selon l'invention, dans l'amélioration de la production de NADPH et donc dans l'amélioration du flux de production d'hydrocortisone.

La production d'hydrocortisone est réalisée dans des souches de *S. cerevisiae* contenant les gènes codant pour les enzymes de la voie de synthèse de l'hydrocortisone (Szczebara et al., 2003, Nature Biotechnology, 21 : 143-149).

| | **µ = 0** | **µ=0,15h⁻¹** | **µ=0,25h⁻¹** |
|---|---|---|---|
| Δ(ZTA1,PGl1) | 0,12 | 0,08 | 0,06 |
| Δ(ZTA1,PGl1) TDH1,2,3-NADP dépendant | 0,21 | 0,14 | 0,10 |
| Δ(ZTA1,PGl1) LPD1-NADP dépendant | 0,20 | 0,14 | 0,10 |
| A(ZTA1, PGl1) TDH1,2,3-NADP dépendant LPD1-NADP dépendant | 0,21 | 0,14 | 0,10 |

### Rendements optimaux théoriques de production d'hydrocortisone par des souches de S. cerevisiae optimisées dans leur capacité de réduction du NADP⁺ (mol par mol de glucose)

Les souches dont les gènes PFK1 et PFK2 sont délétés sont incapables de produire de l'hydrocortisone, voire ne sont pas viables. Ceci est dû au fait que la production d'hydrocortisone est davantage limitée par la demande en carbone que par le besoin en NADPH. Une solution consiste à permettre une légère expression d'une activité de type transhydrogénase chez la levure. Cependant, les modélisations montrent que le flux de production d'hydrocortisone ne sera jamais aussi bon que dans le cas d'une délétion du gène PGI1.

Afin d'améliorer encore le rendement optimal théorique des souches optimisées selon l'invention, des modifications supplémentaires peuvent être prises en compte, telles que la surexpression d'au moins un gène choisi parmi ZWF, SOL1, SOL2, SOL3, SOL4, GND1, GND2, IDP1, IDE2 et IDP3 et/ou la délétion d'au moins un gène choisi parmi ICL1, DAL7.

### RÉFÉRENCES

- Anderson, E.H. (1946) Growth requirements of virus-resistant mutants of Escherichia coli strain "B", Proc. Natl. Acad. Sci. USA 32:120-128
- Baudin, A.; Ozier-Kalogeropoulos, O.; Denouel, A.; Lacroute,F. and Cullin, C. (1993) A simple and efficient method for direct gene deletion in Saccharomyces cerevisiae,., Nucl. Acids Res. 21, 3329-3330
- Bocanegra, J.A. Scrutton, N.S. ; Perham, R.N. (1993) Creation of an NADP-dependent pyruvate dehydrogenase multienzyme complex by protein engineering. Biochemistly 32 : 2737-2740
- Brachmann CB, Davies A, Cost GJ, Caputo E, Li J, Hieter P, Boeke JD. (1998) Designer deletion strains derived from Saccharomyces cerevisiae S288C: a useful set of strains and plasmids for PCR-mediated gene disruption and other applications. Yeast. 14 :115-32.
- Datsenko, K.A. ; Wanner, B.L. (2000) One-step inactivation of chromosomal genes in Escherichia coli K-12 using PCR products. Proc. Natl. Acad. Sci. USA 97 : 6640-6645
- Miller, 1992 ; A Short Course in Bacterial Genetics: A Laboratory Manual and Handbook for Escherichia coli and Related Bacteria, Cold Spring Harbor Laboratory Press, Cold Spring Harbor, New York
- Sambrook et al. (1989 Molecular cloning : a laboratory manual. 2nd Ed. Cold Spring • Harbor Lab., Cold Spring Harbor, New York
- Schaefer U.; Boos, W.; Takors, R.; Weuster-Botz, D. (1999) Automated sampling device for monitoring intracellular metabolite dynamics., Anal. Biochem. 270 : 88-96
- Wach, A.; Brachat,A.; Pohlmann,R.; and Philippsen, P. (1994) New heterologous modules for classical or PCR-based gene disruptions in Saccharomyces cerevisiae, Yeast 10, 1793-1808, 1994.

## Revendications

1. Souche de microorganisme **caractérisée en ce qu'**elle comprend la limitation d'une ou plusieurs activité(s) oxydant le NADPH par la délétion d'un ou plusieurs gène(s) codant pour une quinone oxidoréductase et/ou une transhydrogénase soluble et **en ce qu'**elle comprend également des modifications permettant de favoriser une ou plusieurs activité(s) enzymatique(s) réduisant le NADP⁺ par la délétion d'un ou plusieurs gène(s) codant pour une phosphoglucose isomérase et/ou une phosphofructokinase.

2. Souche selon la revendication 1, **caractérisée en ce qu'**elle comprend également la modification d'un ou plusieurs gène(s) codant pour une dihydrolipoamide déshydrogénase et/ou une glycéraldéhyde 3-phosphate déshydrogénase, afin qu'elle(s) utilise(nt) préférentiellement le NADP.

3. Souche selon l'une des revendications 1 ou 2, **caractérisée en ce qu'**elle comprend également la surexpression d'un ou plusieurs gène(s) codant pour une glucose 6-phosphate déshydrogénase, une 6-phosphogluconolactonase, une 6-phosphogluconate déshydrogénase, une isocitrate déshydrogénase ou une transhydrogénase membranaire.

4. Souche selon l'une des revendications 1 à 3, **caractérisée en ce qu'**elle comprend également la délétion d'un ou plusieurs gène(s) codant pour une 6-phosphogluconate déshydratase, une malate synthase, une isocitrate lyase ou une isocitrate déshydrogénase kinase/phosphatase.

5. Souche selon l'une des revendications 1 à 4, **caractérisée en ce qu'**elle comprend un ou plusieurs gènes, endogène ou exogène, codant des enzymes impliquées dans la biotransformation d'une molécule d'intérêt.

6. Souche selon l'une des revendications 1 à 5, **caractérisée en ce qu'**elle comprend un ou plusieurs gènes marqueurs de sélection.

7. Souche selon l'une des revendications 1 à 6, **caractérisée en ce qu'**elle est choisie parmi les espèces suivantes : *Aspergillus sp.*, *Bacillus sp.*, *Brevibacterium sp., Clostridium sp.*, *Corynebacterium sp.*, *Escherichia sp.*, *Gluconobacter sp.*, *Penicillium sp.*, *Pichia sp.*, *Pseudomonas sp.*, *Rhodococcus sp.*, *Saccharomyces sp.*, *Streptomyces sp.*, *Xanthomonas sp.* et *Candida sp.*

8. Procédé de préparation des souches optimisées selon l'une des revendications 1 à 7, **caractérisé en ce que** l'on délète un ou plusieurs gène(s) codant pour une quinone oxidoréductase et/ou une transhydrogénase soluble, et on délète un ou plusieurs géne(s) codant pour une phosphoglucose isomérase, une phosphofructokinase, une 6-phosphogluconate déshydratase, une malate synthase, une isocitrate lyase ou une isocitrate déshydrogénase kinase/phosphatase, et/ou on modifie un ou plusieurs gène(s) codant pour une dihydrolipoamide déshydrogénase et/ou une Glycéraldéhyde 3-phosphate déshydrogénase, afin qu'elle(s) utilise(nt) préférentiellement le NADP, ces délétions et modifications étant réalisées par un moyen approprié, et/ou on surexprime un ou plusieurs gène(s) codant pour une glucose 6-phosphate déshydrogénase, une 6-phosphogluoonolactonase, une 6-phosphogluconate déshydrogénase, une isocitrate déshydrogénase ou une transhydrogénase membranaire, soit en transformant la souche avec un vecteur approprié comprenant un ou plusieurs géne(s) codant une ou plusieurs enzyme(s) impliquées dans la biotransformation d'une molécule d'intérêt et/ou un ou plusieurs gène(s) marqueur de sélection, soit en modifiant la force du ou des promoteur(s) endogène(s) contrôlant le ou les gène(s) à surexprimer.

9. Procédé de production d'une molécule d'intérêt dont au moins une des réactions de la voie de biosynthèse est NADPH-dépendante, **caractérisé en ce qu'**il comprend les étapes suivantes :
a) mise en culture des microorganismes optimisés selon l'une des revendications 1 à 7 dans un milieu de culture approprié favorisant leur croissance et comprenant les substances nécessaires pour la réalisation de la biotransformation par fermentation ou biotransfonmation, à l'exception du NADPH, et
b) extraction de la molécule d'intérêt du milieu et le cas échéant purification.

10. Procédé selon la revendication 9, **caractérisé en ce que** la molécule d'intérêt est choisie parmi les acides aminés, les vitamines, les stérols, les flavonoides, les acides gras, les acides organiques, les polyols et les hydroxyesters.

## Claims

1. A strain of a micro-organism **characterized in that** one or more of its NADPH-oxidizing activities have been limited by the deletion of one or more genes coding for a quinone oxidoreductase and/or a soluble transhydrogenase and **in that** it has also undergone modifications that favour one or more of its NADP⁺-reducing enzyme activities by the deletion of one or more genes coding for a phosphoglucose isomerase and/or a phosphofructokinase.

2. A strain according to Claim 1 **characterized in that** it has also undergone the modification of one or more genes coding for a dihydrolipoamide dehydrogenase and/or a glyceraldehyde 3-phosphate dehydrogenase so as to cause it to utilize NADP preferentially.

3. A strain according to either of Claims 1 and 2 **characterized in that** it also overexpresses one or more genes coding for a glucose 6-phosphate dehydrogenase, or a 6-phosphogluconolactonase, or a 6-phosphogluconate dehydrogenase, or an isocitrate dehydrogenase or a membrane-bound transhydrogenase.

4. A strain according to any of Claims 1 to 3 **characterized in that** it has also undergone the deletion of one or more genes coding for a 6-phosphogluconate dehydratase, or a malate synthase, or an isocitrate lyase or an isocitrate dehydrogenase kinase/phosphatase.

5. A strain according to any of Claims 1 to 4, **characterized in that** it comprises one or more endogenous or exogenous genes coding for enzymes involved in the biotransformation of a substance of interest.

6. A strain according to any of Claims 1 to 5, **characterized in that** it comprises one or more selection marker genes.

7. A strain according to any of Claims 1 to 6 **characterized in that** it is selected among the following species : *Aspergillus* sp., *Bacillus* sp., *Brevibacterium* sp., *Clostridium* sp., *Corynebacterium* sp., *Escherichia* sp., *Gluconobacter* sp., *Penicillium* sp., *Pichia* sp., *Pseudomonas* sp., *Rhodococcus* sp., *Saccharomyces* sp., *Streptomyces* sp., *Xanthomonas* sp. or *Candida* sp.

8. A method for the preparation of strains optimized according to any of Claims 1 to 7 **characterized in that** it involves the deletion of one or more genes coding for a quinone oxidoreductase and/or a soluble transhydrogenase, and the deletion of one or more genes coding for a phosphoglucose isomerase, or a phosphofructokinase, or a 6-phosphogluconate dehydratase, or a malate synthase, or an isocitrate lyase or an isocitrate dehydrogenase kinase/phosphatase, and/or the modification of one or more genes coding for a dihydrolipoamide dehydrogenase and/or a glyceraldehyde 3-phosphate dehydrogenase, so as to cause it to utilize NADP preferentially, which deletions and modifications are carried out by appropriate means, and/or the overexpression of one or more genes coding for a glucose 6-phosphate dehydrogenase, or a 6-phosphogluconolactonase, or a 6-phosphogluconate dehydrogenase, or an isocitrate dehydrogenase or a membrane-bound transhydrogenase, either by transforming the strain with an appropriate vector containing one or more genes coding for one or more enzymes involved in the biotransformation of a substance of interest and/or one or more selection marker genes, either by modifying the strength of the endogenous promoter(s) controlling the gene(s)to be overexpressed.

9. A method for the production of a substance of interest formed by a biosynthesis route of which at least one step is NADPH-dependent, **characterized in that** it comprises the following steps:
a) growth in culture of micro-organisms optimized according to any of Claims 1 to 7 in an appropriate culture medium that favours their growth and contains substances necessary for carrying out biotransformations by fermentation or bioconversion, except NADPH, and
b) extraction of the substance of interest from the medium and if necessary its purification.

10. A method according to Claim 9 **characterized in that** the substance of interest is an amino acid, or a vitamin, or a sterol, or a flavonoid, or a fatty acid, or an organic acid, or a polyol or a hydroxyester.

## Patentansprüche

1. Mikroorganismusstamm, **dadurch gekennzeichnet, dass** er die Einschränkung einer oder mehrerer NADPH oxidierender Aktivität(en) durch die Deletion eines oder mehrerer Gene umfasst, welche(s) eine Chinon-Oxidoreduktase und/oder eine lösliche Transhydrogenase codiert/codieren, und dass er auch Modifikationen umfasst, die es erlauben, eine oder mehrere enzymatische NADP⁺ reduzierende Aktivität(en) durch die Deletion eines oder mehrerer Gene, welche(s) eine Phosphoglucose-Isomerase und/oder eine Phosphofructokinase codiert/codieren, zu begünstigen.

2. Mikroorganismusstamm gemäß Anspruch 1, **dadurch gekennzeichnet, dass** er auch die Modifikation eines oder mehrerer Gene umfasst, welche(s) eine Dihydrolipoamid-Dehydrogenase und/oder eine Glyceraldehyd-3-Phosphat-Dehydrogenase codiert/codieren, damit es/sie vorzugsweise das NADP verwendet/verwenden.

3. Mikroorganismusstamm gemäß einem der Ansprüche 1 oder 2, **dadurch gekennzeichnet, dass** er auch die Überexpression eines oder mehrerer Gene umfasst, welche(s) eine Glukose-6-Phosophat-Dehydrogenase, eine 6-Phosphogluconolactonase, eine 6-Phosphogluconat-Deyhdrogenase, eine Isocitrat-Dehydrogenase oder eine membranständige Transhydrogenase codiert/codieren.

4. Mikroorganismusstamm gemäß einem der Ansprüche 1 bis 3, **dadurch gekennzeichnet, dass** er auch die Deletion eines oder mehrerer Gene umfasst, welche(s) eine 6-Phosphogluconat-Dehydratase, eine Malatsynthase, eine Isocitratlyase oder eine Isocitrat-Dehydrogenase-Kinase/Phosphatase codiert/codieren.

5. Mikroorganismusstamm gemäß einem der Ansprüche 1 bis 4, **dadurch gekennzeichnet, dass** er ein oder mehrere Gene, endogen oder exogen, umfasst, welche(s) Enzyme codiert/codieren, die an der Biotransformation eines Moleküls von Interesse beteiligt sind.

6. Mikroorganismusstamm gemäß einem der Ansprüche 1 bis 5, **dadurch gekennzeichnet, dass** er ein oder mehrere Selektionsmarkergen(e) umfasst.

7. Mikroorganismusstamm gemäß einem der Ansprüche 1 bis 6, **dadurch gekennzeichnet, dass** er aus den folgenden Arten ausgewählt ist: *Aspergillus sp.*, *Bacillus sp.*, *Brevibacterium sp.*, *Clostridium sp.*, *Corynebacterium sp.*, *Escherichia sp.*, *Gluconobacter sp.*, *Penicillium sp.*, *Pichia sp.*, *Pseudomonas sp.*, *Rhodococcus sp.*, *Saccharomyces sp.*, *Streptomyces sp.*, *Xanthomonas sp.* und *Candida sp.*

8. Verfahren zur Herstellung der optimierten Stämme gemäß einem der Ansprüche 1 bis 7, **dadurch gekennzeichnet, dass** ein oder mehrere Gen(e) deletiert wird/werden, welche(s) eine Chinon-Oxidoreduktase und/oder eine lösliche Transhydrogenase codiert/codieren, und dass ein oder mehrere Gen(e) deletiert wird/werden, welche(s) eine Phosphoglucose-Isomerase, eine Phosphofructokinase, eine 6-Phosphogluconat-Dehydratase, eine Malatsynthase, eine Isocitratlyase oder eine Isocitrat-Dehydrogenase-Kinase/Phosphatase codiert/codieren, und/oder ein oder mehrere Gen(e) modifiziert wird/werden, welche(s) eine Dihydrolipoamid-Dehydrogenase und/oder eine Glyceraldehyd-3-Phosphat-Dehydrogenase codiert/codieren, damit es/sie vorzugsweise das NADP verwendet/verwenden, wobei diese Deletionen und Modifikationen mit einem geeigneten Mittel durchgeführt werden, und/oder dass ein oder mehrere Gen(e) überexprimiert wird/werden, welche(s) eine Glukose-6-Phosophat-Dehydrogenase, eine 6-Phosphogluconolactonase, eine 6-Phosphogluconat-Dehydrogenase, eine Isocitrat-Dehydrogenase oder eine membranständige Transhydrogenase codiert/codieren, entweder durch Transformation des Stammes mit einem geeigneten Vektor, der ein oder mehrere Gen(e) umfasst, die ein oder mehrere Enzym(e), die an der Biotransformation eine Moleküls von Interesse beteiligt sind, und/oder ein oder mehrere Selektionsmarkergen(e) codiert/codieren, oder durch Modifikation der Stärke des oder der endogenen Promotors/Promotoren, welche(r) das oder die zu überexprimierende(n) Gen(e) kontrolliert/kontrollieren.

9. Verfahren zur Herstellung eines Moleküls von Interesse, bei dem zumindest eine der Reaktionen des Biosynthesewegs NADPH-abhängig ist, **dadurch gekennzeichnet, dass** es die folgenden Schritte umfasst:
a) Züchten von optimierten Mikroorganismusstämmen gemäß einem der Ansprüche 1 bis 7 in einem Kulturmedium, das geeignet ist, ihr Wachstum zu begünstigen und das die Substanzen umfasst, die für die Durchführung der Biotransformation durch Fermentation oder Biotransformation notwendig sind, mit Ausnahme von NADPH, und
b) Extraktion des Moleküls von Interesse aus dem Medium und gegebenenfalls Aufreinigung.

10. Verfahren gemäß Anspruch 9, **dadurch gekennzeichnet, dass** das Molekül von Interesse ausgewählt ist aus Aminosäuren, Vitaminen, Sterolen, Flavonoiden, Fettsäuren, organischen Säuren, Polyolen und Hydroxyestern.
